# FASCICULE DE BREVET EUROPEEN

(11) **EP 3 099 255 B1**
(45) Date de publication et mention de la délivrance du brevet: **22.11.2017**
(21) Numéro de dépôt: 15705348.9
(22) Date de dépôt: 29.01.2015
(51) Int. Cl.: A61B 17/34, A61B 17/72, A61B 17/00

(54) **INSTRUMENTATION MEDICALE POUR L'IMPLANTATION DE BROCHES**
MEDIZINISCHES INSTRUMENT ZUM IMPLANTIEREN VON STIFTEN
MEDICAL INSTRUMENT FOR IMPLANTING PINS

(30) Priorité: 30.01.2014 FR 1400246
(43) Date de publication de la demande: 07.12.2016
(73) Titulaire: Persat, Caroline, 1206 Genève (CH)
(72) Inventeur: Persat, Caroline, 1206 Genève (CH)
(74) Mandataire: Thibault, Jean-Marc
(86) Numéro de dépôt international: PCT/FR2015/050216
(87) Numéro de publication internationale: WO 2015/114268

(56) Documents cités:
- US-A1- 2007 012 816
- US-A1- 2008 033 432
- US-A1- 2013 324 997
- US-B1- 6 309 396

## Description

La présente invention concerne le domaine technique des dispositifs adaptés pour l'implantation de broches dans des os longs pour rétablir la résistance mécanique desdits os.

L'objet de l'invention trouve une application particulièrement avantageuse dans le traitement des tumeurs des os longs qui affectent plus ou moins la résistance mécanique des os touchés par de telles tumeurs.

Dans le domaine de la chirurgie orthopédique, il est connu divers appareils pour la mise en place de vis canulés afin par exemple de réduire une fracture par ostéosynthèse. Dans de nombreux cas et en particulier pour des os affectés de tumeurs, l'implantation de vis n'apparaît pas envisageable compte tenu de la détérioration de la résistance mécanique présentée par de tels os.

Dans le domaine de la stabilisation intervertébrale, le document US 2008/0033432 décrit une instrumentation médicale comportant un trocart de pénétration vertébrale équipé d'un fourreau de passage pour un tube d'introduction pour une tige implantable incurvée, Cette tige implantable est montée de façon amovible à l'aide d'une liaison baïonnette à une tige de commande permettant dans un premier temps, de faire sortir la tige implantable en dehors du tube d'introduction et dans un deuxième temps, de désolidariser la tige de commande par rapport à la tige implantable pour permettre son retrait.

Un tel équipement ne permet pas d'assurer un positionnement correct de la tige implantable. En effet, cette dernière qui est poussée hors du tube d'introduction peut suivre divers trajets en fonction du milieu rencontré.

La demande de brevet US 2013/0324997 décrit une instrumentation permettant de placer un moyen de traitement dans une vertèbre en suivant une trajectoire d'implantation incurvée. Cette instrumentation présente les mêmes inconvénients que ceux du document US 2008/0033432 dans la mesure où le moyen de traitement n'est pas placé directement dans sa position définitive par l'instrumentation.

L'objet de la présente invention vise à proposer une nouvelle instrumentation médicale conçue pour permettre de rétablir la résistance mécanique des os longs, par l'implantation de broches positionnées de manière précise et ne dégradent pas la résistance mécanique desdits os.

Pour atteindre un tel objectif, l'instrumentation médicale pour la mise en place de broches dans un os long selon l'invention présente chacune, une longueur déterminée et un diamètre déterminé.

Selon l'invention, l'instrumentation médicale comporte :
- au moins une broche rectiligne présentant une longueur déterminée et un diamètre déterminé,
- un trocart d'introduction comportant un fourreau de guidage présentant une extrémité distale pointue et une extrémité proximale de préhension, un mandrin amovible étant monté coulissant à l'intérieur du fourreau, à partir de son extrémité proximale,
- un guide pour une broche comportant un tube souple présentant un diamètre externe inférieure au diamètre interne du fourreau du trocart pour être introduit dans le fourreau à partir de son extrémité proxirnale, après le retrait du mandrin, le tube souple possédant entre son extrémité distale et son extrémité proximale, une longueur supérieure à la longueur de la broche et un diamètre interne inférieur au diamètre de la broche pour présenter un jeu de fonctionnement assurant la translation de la broche à l'intérieur du tube souple, le tube souple étant réalisé en un matériau malléable, le diamètre interne du tube souple et le diamètre de la broche étant compris entre 0,5 et 2 mm,
- un positionneur pour une broche comportant un mandrin possédant un diamètre externe inférieur au diamètre interne du tube souple pour être introduit et coulisser à l'intérieur du tube souple, le mandrin présentant une extrémité distale formant une surface de poussée pour la broche en vue de positionner l'extrémité distale de la broche au niveau de l'extrémité distale du tube souple, le mandrin délimitant extérieurement une surface de guidage pour le tube souple sur une longueur au moins égale à la longueur du tube souple.

De plus, l'instrumentation médicale selon l'invention peut présenter en outre en combinaison au moins l'une et/ou l'autre des caractéristiques additionnelles suivantes :
- le mandrin du positionneur délimite extérieurement une surface de guidage pour le tube souple sur une longueur égale à la longueur du tube souple,
- le mandrin du positionneur est équipé d'un indicateur monté coulissant sur !e mandrin pour repérer le degré d'enfoncement du mandrin à l'intérieur du tube souple pour positionner l'extrémité distale de la broche au niveau de l'extrémité distale du tube souple,
- la surface de guidage du mandrin possède entre l'indicateur et l'extrémité proximale, une longueur égale au moins à la longueur d'une broche,
- le mandrin du positionneur est équipé en tant qu'indicateur d'une marque ou d'un curseur monté coulissant sur le mandrin pour repérer le degré d'enfoncement du mandrin à l'intérieur du tube souple pour positionner l'extrémité distale de la broche au niveau de l'extrémité distale du tube souple, lors de sa mise en butée sur l'extrémité proximale du tube souple,
- le mandrin du positionneur est pourvu au niveau de l'extrémité proximale de la surface de guidage, d'une butée d'arrêt pour le tube souple,
- le guide pour une broche comporte une crosse d'appui montée amovible sur le guide pour libérer l'accès à l'extrémité proximale du tube souple,
- chaque broche est pourvue d'une extrémité distale et d'une extrémité proximale, délimitées par au moins trois facettes d'orientation différentes se raccordant à une partie d'extrémité émoussée,
- le fourreau du trocart présente une extrémité distale avec une face biseautée terminée en pointe.
   La **Figure 1** est une vue de face d'un trocart d'introduction faisant partie de l'instrumentation médicale conforme à l'invention.
   Les **Figures 1A** et **1B** sont des vues respectivement de profil et de dos du trocart illustré à la **Fig**. **1**.
   La **Figure 2** est une vue d'un guide pour une broche faisant partie de l'instrumentation médicale conforme à l'invention.
   La **Figure 3** est une vue d'un positionneur pour une broche faisant partie de l'instrumentation médicale conforme à l'invention.
   La **Figure 4** est une vue d'une broche faisant partie de l'instrumentation médicale conforme à l'invention.
   La **Figure 5** est une vue illustrant la mise en place d'un trocart d'introduction faisant partie de instrumentation médicale conforme à l'invention.
   La **Figure 6** est une vue illustrant le retrait du mandrin du trocart d'introduction faisant partie de l'instrumentation médicale conforme à l'invention.
   La **Figure 7** est une vue illustrant la mise en place du guide broche dans le trocart d'introduction.
   La **Figure 8** est une vue illustrant le retrait de la crosse du guide broche.
   La **Figure 9** est une vue illustrant la mise en place d'une broche dans le guide broche.
   La **Figure 10** est une vue illustrant l'introduction d'un mandrin d'un positionneur pour une broche dans le guide broche.
   La **Figure 11** est une vue illustrant la mise en place d'une broche dans l'os par le retrait du guide broche.

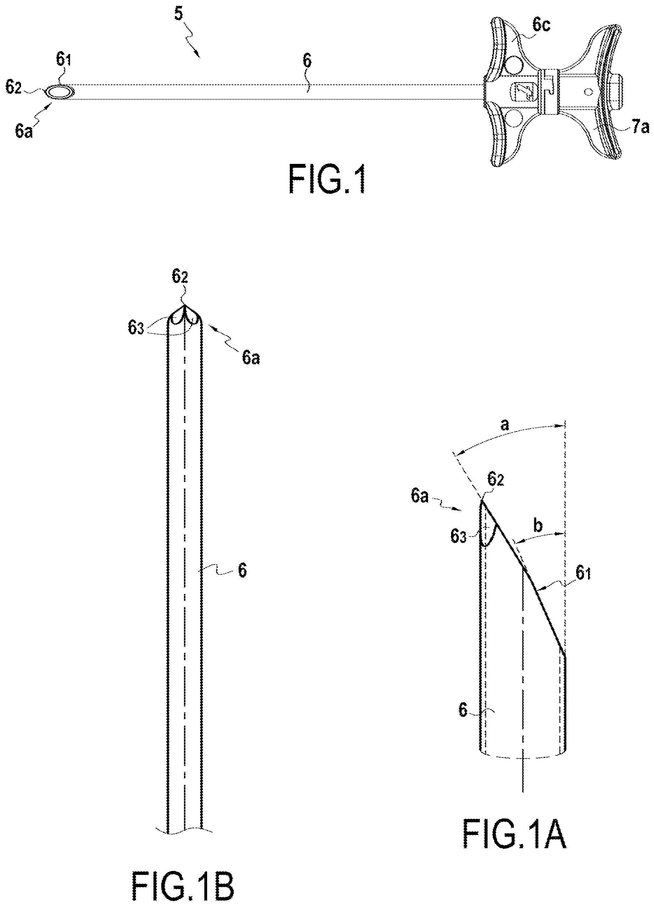

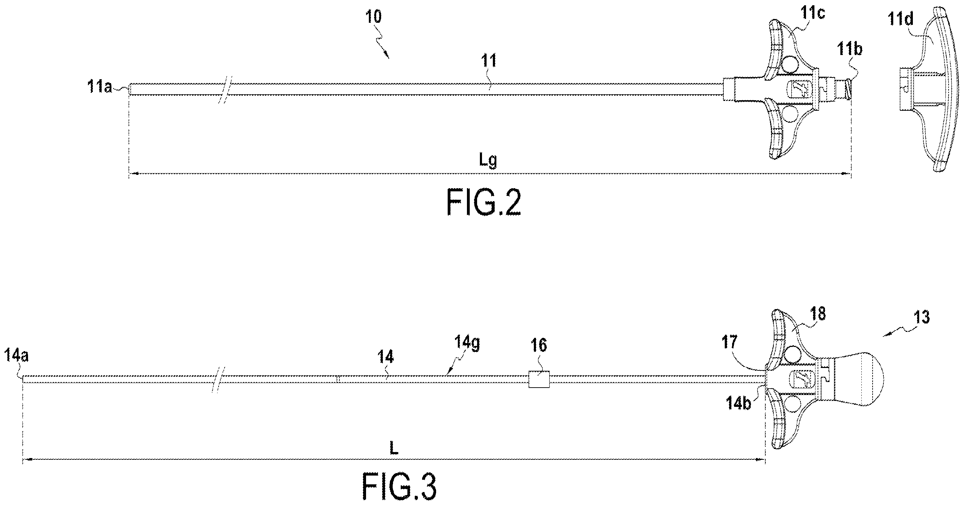

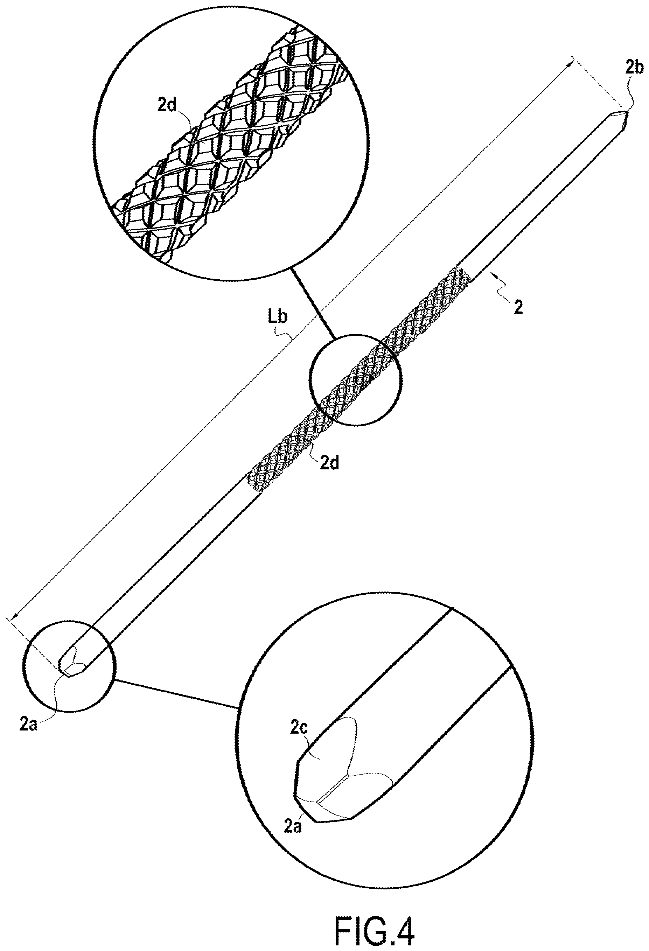

Tel que cela ressort des **Fig. 1 à 4**, l'objet de l'invention concerne une instrumentation médicale **1** pour la mise en place d'au moins une et d'une manière générale de plusieurs broches **2** dans un os long **3** tel que par exemple le fémur, le tibia, le radius ou le cubitus. Chaque broche **2** présente entre une extrémité distale **2a** et une extrémité proximale **2b,** une longueur déterminée **Lb** comprise entre 5 et 25 cm, choisie pour s'adapter à la reconstruction de la résistance mécanique de l'os (**Fig**. **4**). Chaque broche **2** qui est droite ou rectiligne possède un diamètre compris entre 2 et 5 mm choisi pour s'adapter à la résistance mécanique de la reconstruction. Les broches **2** peuvent être réalisées en tout matériau de grade médical implantable dans le corps humain.

Avantageusement, l'extrémité distale **2a** et l'extrémité proximale **2b** des broches comportent chacune au moins trois facettes d'orientation différentes **2c** se raccordant à une partie commune d'extrémité émoussée ou arrondie. Avantageusement, les facettes **2c** présentent chacune par rapport à l'axe longitudinal de la broche une angulation comprise entre 15 et 45° tandis que la partie commune d'extrémité présente un rayon de courbure minimale supérieure ou égale à 1 mm. Une telle configuration des extrémités des broches **2** permet aux broches de glisser l'une sur l'autre en cas d'implantation multiple de broches comme cela sera expliqué dans la suite de la description. Selon une variante avantageuse de réalisation, chaque broche **2** comporte une partie centrale **2d**, munie d'un traitement de surface lui donnant une rugosité adaptée pour assurer la fixation de la broche dans une masse de ciment.

L'instrumentation médicale **1** comporte aussi un trocart d'introduction **5** comportant un fourreau de guidage **6** présentant une extrémité distale pointue **6a** de préférence en forme de biseau pour présenter un haut pouvoir de pénétration dans l'os (**Fig**. **1**, **5** et **6**).

Tel que cela ressort plus précisément des **Fig**. **1**, **1A** et **1B**, l'extrémité distale **6a** du fourreau **6** du trocart **5** présente une face biseautée **6₁** dont la partie antérieure **6₂** est pointue. Avantageusement, la face biseautée **6₁** s'étend à partir de la partie antérieure **6₂**, selon une annulation **a** de l'ordre de 30° par rapport à l'axe du fourreau **6.** De préférence, la face biseautée **6₁** s'étend à l'opposé de la partie antérieure **6₂**, selon une annulation **b** inférieure à l'angulation **a** (par exemple de l'ordre de 25°). De préférence, l'extrémité distale **6a** du fourreau **6** comporte à l'opposé de la face biseauté **6₁**, deux facettes biseautées **6₃** se raccordant au niveau de la partie antérieure pointue **6₂**.

Le fourreau de guidage **6** comporte à l'opposé de l'extrémité distale **6a**, une extrémité proximale de préhension **6b** équipé d'un corps d'appui **6c** pour les doigts s'étendant transversalement de part et d'autre du fourreau **6.** Le trocart **5** comporte également un mandrin **7** monté coulissant à l'intérieur du fourreau **6**, à partir de son extrémité proximale **6b**. Classiquement, le mandrin **7** qui est amovible par rapport au fourreau de guidage **6** est pourvu à son extrémité proximale, d'une crosse de préhension **7a**. Lorsque le mandrin **7** est retiré du fourreau **6**, alors l'extrémité proximale **6b** du fourreau est dégagée.

L'instrumentation médicale **1** comporte également un guide **10** pour une broche **2** comportant un tube souple **11** présentant un diamètre externe inférieur au diamètre interne du fourreau **6** du trocart pour être introduit dans le fourreau **6** à partir de son extrémité proximale **6b**, après bien entendu, le retrait du mandrin **7** du trocart. Le tube souple **11** est réalisé en un matériau malléable tel qu'en un alliage métallique comme en acier inoxydable ou en acier composite hyper trempé. Ce tube souple **11** présente un caractère malléable dans le sens où comme cela sera expliqué dans la suite de la description ce tube est déformable et apte à conserver la forme déformée prise.

Le tube souple **11** possède une extrémité distale **11a** par exemple droite adaptée pour servir de repérage pour la mise en place des broches comme cela sera expliqué dans la suite de la description. Le tube souple **11** comporte entre son extrémité distale **11a** et son extrémité proximale **11b**, une longueur **Lg** supérieure à la longueur **Lb** de la broche et un diamètre interne supérieur au diamètre de la broche pour présenter un jeu de fonctionnement assurant la translation de la broche 3 l'intérieur du tube souple **11**. Avantageusement, la différence entre le diamètre interne du tube souple **11** et le diamètre de la broche **2,** c'est-à-dire le jeu de fonctionnement est compris entre 0,5 et 2 mm,

Avantageusement, le tube souple **11** est pourvu à son extrémité proximale **11b,** d'un corps d'appui **11c** pour les doigts sur lequel est montée de manière amovible, une crosse d'appui **11d** dont le retrait permet de libérer l'accès à l'extrémité proximale **11b** du tube souple **11**.

L'instrumentation médicale **1** comporte également un positionneur **13** pour une broche **2** comportant un mandrin **14** possédant un diamètre externe inférieur au diamètre interne du tube souple **11** pour être introduit et coulisser à l'intérieur du tube souple **11** en vue de positionner l'extrémité distale **2a** de la broche **2** au niveau de l'extrémité distale **11a** du tube souple. Le mandrin **14** présente une extrémité distale **14a** formant une surface de poussée pour la broche **2.**

Selon une caractéristique de l'invention, le mandrin **14** du positionneur **13** délimite extérieurement, une surface de guidage **14g** pour le tube souple **11** lorsque le guide **10** est à retirer après le positionnement définitif de la broche **2**. Cette surface de guidage **14g** du mandrin **14** du positionneur **13** possède une longueur **L** au moins égale à la longueur d'une broche **2.** La langueur **L** de la surface de guidage **14g** est prise entre l'extrémité distale **14a** du mandrin **14** du positionneur **13** et son l'extrémité proximale **14b**.

Selon une variante avantageuse de réalisation, le mandrin **14** du positionneur **13** délimite extérieurement une surface de guidage **14g** pour le tube souple sur une longueur **L** sensiblement égale à la longueur **Lb** du tube souple **11.** En effet, lorsque le mandrin **14** du positionneur **13** a placé l'extrémité distale **2a** de la broche au niveau de l'extrémité distale du tube souple **11**, alors le mandrin **14** pénètre à l'intérieur du tube souple selon une mesure égale à la longueur **Lg** du tube souple **11** diminuée de la langueur **Lb** de la broche. Le mandrin **14** doit présenter en dehors du tube souple **11,** une surface de guidage **14g** de longueur au moins égale à la longueur **Lb** de la broche pour permettre le dégagement du tube souple **11** par rapport à la broche **2.**

Selon une variante préférée de réalisation, le mandrin **14** du positionneur **13** est équipé d'un indicateur **16** du degré d'enfoncement du mandrin **14** du positionneur **13,** à l'intérieur du tube souple **11** pour positionner l'extrémité distale **2a** de la broche **2** au niveau de l'extrémité distale **11a** du tube souple. Cet indicateur **16** peut être formé par un signe ou une marque réalisé sur le mandrin **14** ou par un curseur monté coulissant sur le mandrin pour permettre de régler !e degré d'enfoncement du mandrin. De préférence, l'indicateur **16** est réalisé ou monté de manière à être positionné au niveau de l'extrémité proximale **11b** du guide **10** lorsque le mandrin **14a** a positionné l'extrémité distale **2a** de la broche **2** au niveau de l'extrémité distale **11a** du tube souple **11**.

Dans l'exemple illustré sur les dessins, l'indicateur **16** est un curseur monté coulissant sur le mandrin **14** pour repérer le degré d'enfoncement du mandrin à l'intérieur du tube souple **11** pour positionner l'extrémité distale **2a** de la broche **2** au niveau de l'extrémité distale **11a** du tube souple, lors de sa mise en butée sur l'extrémité proximale **11b** du tube souple **11**. Comme cela sera expliqué dans la suite de la description, ce curseur **16** est déplacé en translation par le guide **10** lors de son opération de retrait.

Avantageusement, le mandrin **14** du positionneur **13** est pourvu au niveau de l'extrémité proximale **14b** de la surface de guidage **14,** d'une butée d'arrêt **17** pour le tube souple **11**. Par exemple, la butée d'arrêt **17** est réalisée sur une poignée de préhension **18** équipant le mandrin **14.** Dans le cas où le positionneur **13** est pourvu d'un curseur **16** monté coulissant sur le mandrin **14** du positionneur **13**, alors la longueur **L** de la surface de guidage **14g** est prise entre l'extrémité distale **14a** du mandrin **14** du positionneur **13** et le curseur **16** occupant sa position de butée sur la butée d'arrêt **17.**

En d'autres termes, la distance entre l'indicateur **16** du degré d'enfoncement et l'extrémité proximale **14b** du mandrin c'est-à-dire de la butée d'arrêt **17** est égale au moins à la longueur **Lb** d'une broche **2** et de préférence égale à la longueur d'une broche **2**.

La mise en oeuvre de l'instrumentation médicale **1** selon l'invention découle directement de la description qui précède.

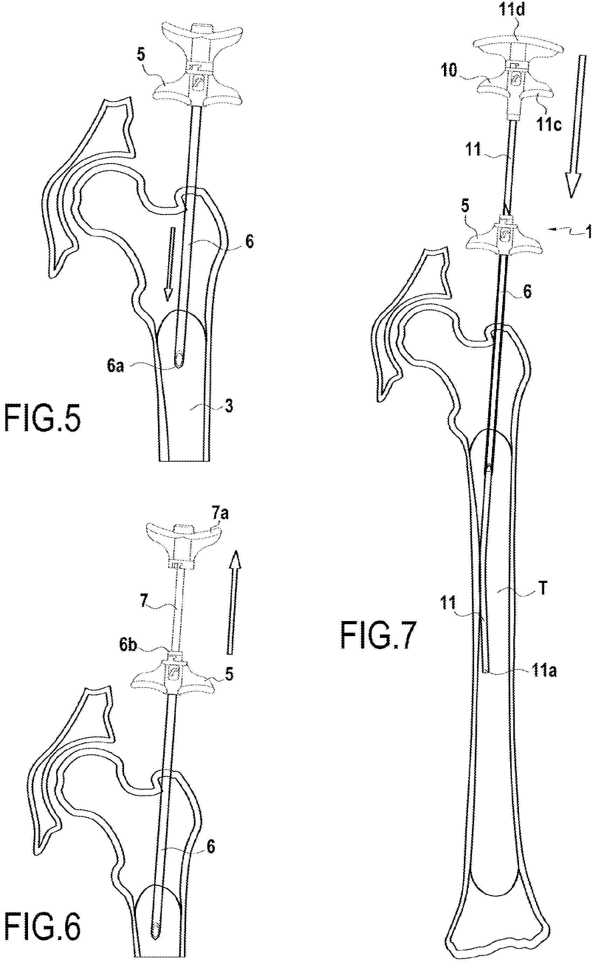

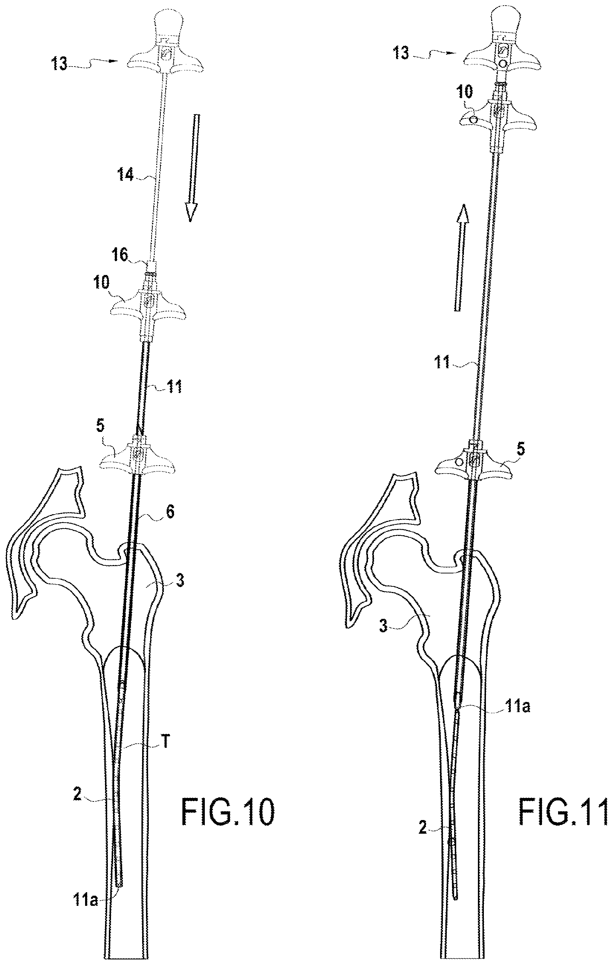

L'instrumentation médicale **1** selon l'invention permet la mise en place d'au moins une broche **2** dans une zone tumorale **T**, affectant l'os fémoral **3** dans l'exemple illustré aux **Fig. 5** à **11** décrivant les étapes d'implantation. La première étape consiste à mettre en place à partir d'une épiphyse de l'os, le trocart **5.** La voie d'abord est effectuée selon l'axe de l'os long à partir de l'une de ses épiphyses. Cette voie d'abord est transcutanée et guidée radiologiquement. Le trocart **5** pénètre dans l'os en franchissant la barrière osseuse de sorte que l'extrémité distale **6a** du trocart **5** atteigne la cavité médullaire de l'os (**Fig**. **5**).

Le mandrin **7** du trocart **5** est ensuite retiré comme illustré à la **Fig. 6****.**

Ensuite, le guide **10** est introduit dans le trocart **5** par l'insertion de l'extrémité distale **11a** du tube souple à l'intérieur du fourreau de guidage **6,** de manière que l'extrémité distale **11a** du tube souple **11** se trouve positionnée au point d'implantation de l'extrémité distale de la broche (**Fig. 7**). Il est à noter que la souplesse du tube souple **11** permet de suivre le trajet imposé par la corticale interne de l'os. En d'autres termes, en raison du caractère malléable du tube souple **11,** ce dernier se conforme au trajet plus ou moins sinueux imposé par la corticale interne de l'os. La position définitive de l'extrémité distale **11a** du tube souple **11** est réalisée par imagerie radiologique. Il est à noter que si l'extrémité distale **11a** du tube souple **11** n'occupe pas la position souhaitée pour l'extrémité distale de la broche **2**, alors le guide **10** est de nouveau manipulé par exemple selon un trajet différent pour amener l'extrémité distale **11a** du tube souple à occuper la position souhaitée.

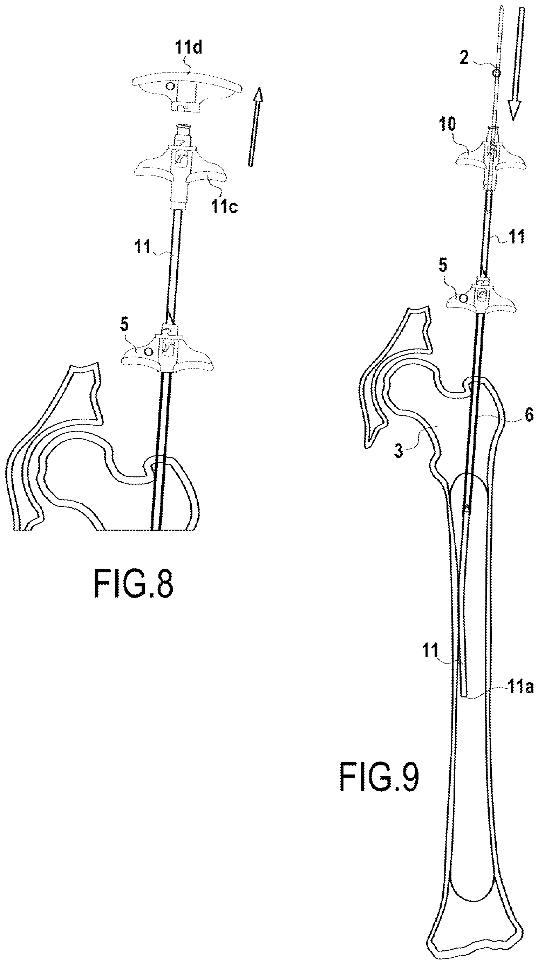

La méthode consiste ensuite à retirer la crosse d'appui **11d** pour permettre de libérer l'accès à l'extrémité proximale **11b** du tube souple (**Fig. 8**). Le procédé consiste à introduire dans le tube souple **11** maintenu en position fixe par rapport au trocart, à partir de son extrémité proximale **11b**, une broche **2** (**Fig. 9**) puis le positionneur **13** (**Fig, 10**). Le mandrin **14** du positionneur **13** est déplacé à l'intérieur du tube souple **11** pour pousser la broche **2** et amener l'extrémité distale **2a** de la broche **2** au niveau de l'extrémité distale **11a** du tube souple **11**. Dans le cas où le mandrin **14** est équipé d'un indicateur ou d'un curseur **16** alors ce dernier est positionné sur le mandrin **14** à une distance de l'extrémité distale **14a** du mandrin qui est égale à la longueur **Lg** du tube souple **11** diminuée de la longueur **Lb** de la broche. Ainsi, le mandrin **14** est poussé pour déplacer la broche **2** jusqu'à ce que soit l'indicateur **16** se trouve situé au niveau de l'extrémité proximale **11b** du tube souple **11** ou soit le curseur **16** vient en butée sur l'extrémité proximale **11b** du tube souple **11**. Dans cette position de butée, la course de pénétration du mandrin **14** est arrêtée car l'extrémité distale **2a** de la broche **2** coïncide avec l'extrémité distale **11a** du tube souple **11**.

La méthode consiste ensuite à maintenir en position fixe le positionneur **13** de broche, et à exercer simultanément une traction sur le guide **10** pour retirer le tube souple **11** par rapport à la broche **2** (**Fig**. **11**). Dans la mesure où le mandrin **14** du positionneur **13** est maintenu en position fixe, la broche **2** reste en position pendant le retrait du tube souple **11** par rapport à la broche **2**.

Il est à noter que le curseur **16** est monté sur le mandrin **14** avec une possibilité de déplacement lorsqu'il est soumis à l'effort de poussée du guide **10** lors du retrait de ce dernier. Le curseur **16** agit ainsi comme un fretin sur le guide **10**.

Avantageusement, le guide **10** est translaté sur une course égale à la longueur **Lb** de la bronche. La fin de la course de retrait du guide **10** correspond avantageusement à la mise en butée sur la butée d'arrêt **17**, de l'extrémité proximale **11b** du tube souple **11**. En effet, la surface de guidage **14g** du mandrin **14** possède entre l'indicateur **16** et l'extrémité proximale **14b** du mandrin équipée de la butée d'arrêt **17,** une longueur égale à la longueur d'une broche **2**.

Enfin, le guide **10,** le positionneur **13** et le trocart **5** sont retirés. Le procédé tel que décrit ci-dessus est avantageusement recommencé pour implanter une nouvelle broche **2** dans la zone tumorale **T**. A cet effet, le trocart **5** est introduit dans l'épiphyse de l'os selon une direction d'implantation différente de la première implantation. Les étapes pour la mise en place d'une broche décrites ci-dessus sont ensuite renouvelées. Plusieurs broches **2** peuvent ainsi être implantées sous la forme d'un faisceau pour améliorer la résistance mécanique de l'os. La forme en facettes des extrémités distales des broches **2** leur permette de glisser l'une sur l'autre en éliminant le risque que les broches se poussent entre-elles.

Au terme de l'implantation des broches **2**, le procédé consiste à injecter un ciment dans l'os, introduit dans le guide **10** et mis en place à l'aide du mandrin **14** du positionneur **13.** Les broches **2** sont ainsi noyées dans un ciment consolidant l'os long.

L'invention n'est pas limitée aux exemples décrits et représentés car diverses modifications peuvent y être apportées sans sortir de son cadre qui est définit par les revendications.

## Revendications

1. Instrumentation médicale pour la mise en place de broches (**2**) dans un os long (**3**), comportant :
- au moins une broche (**2**) rectiligne présentant, une longueur déterminée et un diamètre déterminé
- un trocart d'introduction (**5**) comportant un fourreau de guidage (**6**) présentant une extrémité distale pointue (**6a**) et une extrémité proximale de préhension, un mandrin amovible (**7**) étant monté coulissant à l'intérieur du fourreau, à partir de son extrémité proximale,
- un guide (**10**) pour une broche (**2**) comportant un tube souple (**11**) présentant un diamètre externe inférieure au diamètre interne du fourreau du trocart pour être introduit dans le fourreau à partir de son extrémité proximale, après le retrait du mandrin, le tube souple (**11**) possédant entre son extrémité distale et son extrémité proximale, une longueur supérieure à la longueur de la broche et un diamètre interne supérieur au diamètre de la broche pour présenter un jeu de fonctionnement assurant la translation de la broche à l'intérieur du tube souple, le tube souple (**11**) étant réalisé en un matériau malléable, le diamètre interne du tube souple (**11**) et le diamètre de la broche (**2**) étant compris entre 0,5 et 2 mm,
- un positionneur (**13**) pour une broche (**2**) comportant un mandrin (**14**) possédant un diamètre externe inférieur au diamètre interne du tube souple (**11**) pour être introduit et coulisser à l'intérieur du tube souple, le mandrin (**14**) présentant une extrémité distale (**14a**) formant une surface de poussée pour la broche (**2**) en vue de positionner l'extrémité distale de la broche au niveau de l'extrémité distale du tube souple, le mandrin (**14**) délimitant extérieurement une surface de guidage (**14g**) pour le tube souple (**11**) sur une longueur au moins égale à la longueur du tube souple.

2. Instrumentation selon la revendication 1, **caractérisée en ce que** le mandrin (**14**) du positionneur (**13**) délimite extérieurement une surface de guidage (**14g**) pour le tube souple sur une longueur égale à la longueur du tube souple.

3. Instrumentation selon la revendication 1 ou 2, **caractérisée en ce que** le mandrin (**14**) du positionneur (**13**) est équipé d'un indicateur (**16**) pour repérer le degré d'enfoncement du mandrin à l'intérieur du tube souple (**11**) pour positionner l'extrémité distale (**2a**) de la broche (**2**) au niveau de l'extrémité distale (**11a**) du tube souple (**11**).

4. Instrumentation selon la revendication 3, **caractérisée en ce que** la surface de guidage (**14g**) du mandrin possède entre l'indicateur (**16**) et l'extrémité proximale (**14b**), une longueur égale au moins à la longueur (**Lb**) d'une broche (**2**).

5. Instrumentation selon la revendication 3 ou 4, **caractérisée en ce que** le mandrin (**14**) du positionneur (**13**) est équipé en tant qu'indicateur (**16**) d'une marque ou d'un curseur monté coulissant sur le mandrin pour repérer le degré d'enfoncement du mandrin à l'intérieur du tube souple (**11**) pour positionner l'extrémité distale (**2a**) de la broche (**2**) au niveau de l'extrémité distale (**11a**) du tube souple (**11**), lors de sa mise en butée sur l'extrémité proximale (**11b**) du tube souple (**11**).

6. Instrumentation selon l'une des revendications 1 à 5, **caractérisée en ce que** le mandrin (**14**) du positionneur (**13**) est pourvu au niveau de l'extrémité proximale de la surface de guidage, d'une butée d'arrêt (**17**) pour le tube souple.

7. Instrumentation selon l'une des revendications 1 à 6, **caractérisée en ce que** le guide (**10**) pour une broche (**2**) comporte une crosse d'appui (**11c**) montée amovible sur le guide pour libérer l'accès à l'extrémité proximale (**11b**) du tube souple.

8. Instrumentation selon l'une des revendications 1 à 7, **caractérisée en ce que** chaque broche (**2**) est pourvue d'une extrémité distale (**2a**) et d'une extrémité proximale (**2b**), délimitées par au moins trois facettes d'orientation différentes se raccordant à une partie d'extrémité émoussée.

9. Instrumentation selon la revendication 1, **caractérisée en ce que** le fourreau (**6**) du trocart (**5**) présente une extrémité distale (**6a**) avec une face biseautée terminée en pointe.

## Patentansprüche

1. Medizinisches Instrument zum Einsetzen von Stiften (2) in einen langen Knochen (3), umfassend
- wenigstens einen geradlinigen Stift (2), der eine bestimmte Länge und einen bestimmten Durchmesser aufweist,
- einen Einführungstrokar (5), der eine Führungshülse (6) mit einem spitzzulaufenden distalen Ende (6a) und einem proximalen Greifende umfasst, wobei ein lösbarer Mandrin (7) innerhalb der Hülse von ihrem proximalen Ende aus verschieblich angebracht ist,
- eine Führung (10) für einen Stift (2), die eine flexible Röhre (11) umfasst, welche einen Außendurchmesser kleiner als der Innendurchmesser der Hülse des Trokars aufweist, um nach Zurückziehen des Mandrins in die Hülse ausgehend von ihrem proximalen Ende eingeführt zu werden, wobei die flexible Röhre (11) zwischen ihrem distalen Ende und ihrem proximalen Ende eine Länge größer als die Länge des Stiftes und einen Innendurchmesser größer als der Durchmesser des Stiftes besitzt, um ein Funktionsspiel aufzuweisen, das das Verschieben des Stiftes innerhalb der flexiblen Röhre sicherstellt, wobei die flexible Röhre (11) aus einem geschmeidigen Material ausgebildet ist, wobei der Innendurchmesser der flexiblen Röhre (11) und der Durchmesser des Stiftes (2) im Bereich zwischen 0,5 bis 2 mm liegen,
- einen Positionierer (13) für einen Stift (2), umfassend einen Mandrin (14), der einen Außendurchmesser kleiner als der Innendurchmesser der flexiblen Röhre (11) besitzt, um in die flexible Röhre eingeführt und darin verschoben zu werden, wobei der Mandrin (14) ein distales Ende (14a) aufweist, das eine Schubfläche für den Stift (2) bildet, um das distale Ende des Stiftes im Bereich des distalen Endes der flexiblen Röhre zu positionieren, wobei der Mandrin (14) außen eine Führungsfläche (14g) für die flexible Röhre (11) über eine Länge, die wenigstens gleich der Länge der flexiblen Röhre ist, begrenzt.

2. Instrument nach Anspruch 1, **dadurch gekennzeichnet, dass** der Mandrin (14) des Positionierers (13) außen eine Führungsfläche (14g) für die flexible Röhre über eine Länge, die gleich der Länge der flexiblen Röhre ist, begrenzt.

3. Instrument nach Anspruch 1 oder 2, **dadurch gekennzeichnet, dass** der Mandrin (14) des Positionierers (13) mit einem Anzeiger (16) versehen ist, um den Eintauchgrad des Mandrins innerhalb der flexiblen Röhre (11) zu erkennen, um das distale Ende (2a) des Stiftes (2) im Bereich des distalen Endes (11a) der flexiblen Röhre (11) zu positionieren.

4. Instrument nach Anspruch 3, **dadurch gekennzeichnet, dass** die Führungsfläche (14g) des Mandrins zwischen dem Anzeiger (16) und dem proximalen Ende (14b) eine Länge, die wenigstens gleich der Länge (Lb) eines Stiftes (2) ist, besitzt.

5. Instrument nach Anspruch 3 oder 4, **dadurch gekennzeichnet, dass** der Mandrin (14) des Positionierers (13) als Anzeiger (16) mit einer Markierung oder einem Schieber, welcher an dem Mandrin verschieblich angebracht ist, versehen ist, um den Eintauchgrad des Mandrins innerhalb der flexiblen Röhre (11) zu erkennen, um das distale Ende (2a) des Stiftes (2), bei seinem In-Anschlag-bringen an dem proximalen Ende (11b) der flexiblen Röhre (11), im Bereich des distalen Endes (11a) der flexiblen Röhre (11) zu positionieren.

6. Instrument nach einem der Ansprüche 1 bis 5, **dadurch gekennzeichnet, dass** der Mandrin (14) des Positionierers (13) im Bereich des proximalen Endes der Führungsfläche mit einem Anschlag (17) für die flexible Röhre versehen ist.

7. Instrument nach einem der Ansprüche 1 bis 6, **dadurch gekennzeichnet, dass** die Führung (10) für einen Stift (2) einen Anlagegriff (11c) umfasst, der an der Führung lösbar angebracht ist, um den Zugang zu dem proximalen Ende (11b) der flexiblen Röhre freizugeben.

8. Instrument nach einem der Ansprüche 1 bis 7, **dadurch gekennzeichnet, dass** jeder Stift (2) mit einem distalen Ende (2a) und mit einem proximalen Ende (2b) versehen ist, die durch wenigstens drei unterschiedliche Ausrichtungsfacetten, welche an einen abgestumpften Endteil anschließen, begrenzt sind.

9. Instrument nach Anspruch 1, **dadurch gekennzeichnet, dass** die Hülse (6) des Trokars (5) ein distales Ende (6a) mit einer abgeschrägten, spitz abschließenden Seite aufweist.

## Claims

1. A medical instrument for putting pins (2) into place in a long borne (3), the instrument comprising:
at least one rectilinear pin (2) presenting a determined length and a determined diameter;
an insertion trocar (5) comprising a guide sheath (6) presenting a pointed distal end (6a) and a proximal grip end, a removal obturator (7) being slideably mounted inside the sheath from its proximal end;
a guide (10) for a pin (2) the guide comprising a flexible tube (11) presenting an outside diameter less than the inside diameter of the sheath of the trocar to enable it to be inserted in the sheath from its proximal end, after the obturator has been removed, the flexible tube (11) presenting between its distal end and its proximal end a length that is greater than the length of the pin and an inside diameter that is greater than the diameter of the pin so as to leave operating clearance enabling the pin to move in translation inside the flexible tube; the flexible tube (11) being made of a malleable material, the inside diameter of the flexible tube (11) and the diameter of the pin (2) being between in the range 0.5 to 2 mm;
a positioner (13) for positioning a pin (2), the positioner comprising an obturator (14) possessing an outside diameter less than the inside diameter of the flexible tube (11) in order to enable it to be inserted in and to slide inside the flexible tube, the obturator (14) presenting a distal end (14a) forming a thrust surface for the pin (2) in order to position the distal end of the pin at the distal end of the flexible tube, the obturator (14) externally defining a guide surface (14g) for the flexible tube (11) over a length not less than the length of the flexible tube.

2. An instrument according to claim 1, **characterized in that** the obturator (14) of the positioner (13) defines externally a guide surface (14g) for the flexible tube over a length equal to the length of the flexible tube.

3. An instrument according to claim 1 or claim 2, **characterized in that** the obturator (14) of the positioner (13) is fitted with an indicator (16) to identify the degree to which the obturator is pushed into the inside of the flexible tube (11) in order to position the distal end (2a) of the pin (2) at the distal end (11a) of the flexible tube (11).

4. An instrument according to claim 3, **characterized in that** the guide surface (14g) of the obturator possesses a length between the indicator (16) and the proximal end (14b) that is equal at least to the length (Lb) of a pin (2).

5. An instrument according to claim 3 or claim 4, **characterized in that** the obturator (14) of the positioner (13) is fitted as an indicator (16) with a mark or with a cursor slidably mounted on the obturator to identify the degree to which the obturator is pushed into the inside of the flexible tube (11) in order to position the distal end (2a) of the pin (2) at the distal end (11a) of the flexible tube (11) while being put into abutment against the proximal end (11b) of the flexible tube (11).

6. An instrument according to any one of claims 1 to 5, **characterized in that** the obturator (14) of the positioner (13) is provided at the proximal end of the guide surface with a stop abutment (17) for the flexible tube.

7. An instrument according to any one of claims 1 to 6, **characterized in that** the guide (10) for a pin (2) includes a bearing cross-bar (11c) removably mounted on the guide in order to release access to the proximal end (11b) of the flexible tube.

8. An instrument according to any one of claims 1 to 7, **characterized in that** each pin (2) is provided with a distal end (2a) and with a proximal end (2b) that are defined by at least three facets of different orientations that come together at a blunted end portion.

9. An instrument according to claim 1, **characterized in that** the sheath (6) of the trocar (5) presents a distal end (6a) with a beveled face terminated in a point.
